# EUROPÄISCHE PATENTSCHRIFT

(11) **EP 4 212 955 B1**
(45) Veröffentlichungstag und Bekanntmachung des Hinweises auf die Patenterteilung: **21.02.2024**
(21) Anmeldenummer: 23161240.9
(22) Anmeldetag: 30.04.2020
(51) Int. Cl.: G03B 33/10

(54) **VORRICHTUNG ZUM FARBABHÄNGIGEN DETEKTIEREN VON BILDINHALTEN SOWIE RECHENVORRICHTUNG UND KRAFTFAHRZEUG MIT EINER SOLCHEN VORRICHTUNG**
DEVICE FOR A COLOR-BASED DETECTION OF IMAGE CONTENTS, COMPUTING DEVICE, AND MOTOR VEHICLE COMPRISING SUCH A DEVICE
DISPOSITIF DE DÉTECTION DE CONTENUS D'IMAGES EN FONCTION DE LA COULEUR, ORDINATEUR ET VÉHICULE À MOTEUR ÉQUIPÉ D'UN TEL DISPOSITIF

(30) Priorität: 03.05.2019 DE 102019206363
(43) Veröffentlichungstag der Anmeldung: 19.07.2023
(62) Teilanmeldung aus: 20724042.5
(73) Patentinhaber: Audi AG, 85045 Ingolstadt (DE)
(72) Erfinder: KLUG, Markus, 85057 Ingolstadt (DE); MOLL, Tobias, 85055 Ingolstadt (DE); SCHEUCHENPFLUG, Johannes, 85107 Baar-Ebenhausen (DE)
(74) Vertreter: Hofstetter, Schurack & Partner

(56) Entgegenhaltungen:
- KR-B1- 101 698 102
- US-A1- 2018 232 048

## Beschreibung

Die Erfindung betrifft eine Vorrichtung zum farbabhängigen Detektieren von Bildinhalten sowie ein Kraftfahrzeug und eine Rechenvorrichtung mit einer solchen Vorrichtung.

Heutzutage gibt es verschiedene Aufnahmesysteme, die über Lichtquellen mit unterschiedlicher Wellenlänge und über Farbfilter eine farbabhängige Aufnahme von Bildinhalten ermöglichen. Dies kann beispielsweise zur Erweiterung des sichtbaren Spektrums verwendet werden, um beispielsweise Oberflächendetails, Krankheiten oder ein biometrisches Identifikationsmerkmal, wie beispielsweise ein Handvenenmuster, zu erkennen.

Nachteilig bei bisherigen Aufnahmesystemen ist es, dass die Lichtquelle nicht mit einer optischen Achse einer Kamera überlagert werden kann, wodurch ein schräge Beleuchtung resultiert und dadurch sich die Position der Bildinhalte verschieben kann, was einer Verschiebung der optischen Achse entspricht. Dieser Umstand kann mit zunehmender Anzahl von Lichtquellen. Eine Möglichkeit des Ausgleichs dieser Verschiebung sind optische Elemente, beispielsweise Prismen, die jedoch einen erhöhten Bauraumbedarf aufweisen.

Aus der DE 10 2016 206 290 A1 ist ein Kamerasystem mit wenigstens einem Kameramodul und wenigstens einer Streulichtfalle bekannt, wobei die wenigstens eine Streulichtfalle, insbesondere an ihrer Innenseite, eine streulichtreduzierende Struktur umfasst, wobei die streulichtreduzierende Struktur wenigstens ein holographisches Element, insbesondere ein Volumenhologramm, umfasst.

Die US 2018/0232048 A1 offenbart einen Objekttracker mit einem Lichtleiter, einer Lichtquelle und einem mit dem Lichtleiter gekoppelten Detektor. Der Lichtleiter ist mit wenigstens einem optischen Gitter ausgestattet. Im Lichtleiter wird Licht der Lichtquelle auf einem ersten optischen Pfad zum Objekt weitergeleitet. Am Objekt reflektiertes Licht wird mittels des Gitters auf einen zweiten optischen Pfad gelenkt und im Lichtleiter zum Detektor transportiert.

Die KR 101 698 102 B1 offenbart eine Vorrichtung zum Steuern eines Fahrzeugs, mit einer ersten Bilderfassungsvorrichtung, die erste Bildinformationen von wenigstens einem Teil eines Benutzers im Inneren des Fahrzeugs erhält und einer zweiten Bilderfassungsvorrichtung, die zweite Bildinformationen von wenigstens einem Teil der Außenseite des Fahrzeugs erhält. Ein Steuergerät erkennt die Form der Hand des Benutzers aus den ersten Bildinformationen und bestimmt ein Zielobjekt, das der Form der Hand entspricht, aus den zweiten Bildinformationen.

Der Erfindung liegt die Aufgabe zugrunde, eine Vorrichtung zum farbabhängigen Detektieren von Bildinhalten bereitzustellen.

Die Aufgabe wird durch die Gegenstände der unabhängigen Patentansprüche gelöst. Vorteilhafte Weiterbildungen der Erfindung sind durch die abhängigen Patentansprüche, die folgende Beschreibung sowie die Figuren offenbart.

Durch die Erfindung ist eine Vorrichtung zum farbabhängigen Detektieren von Bildinhalten bereitgestellt. Die Vorrichtung ist ausgestattet mit einem Trägermedium, welches als Lichtleiter zum Übertragen von eingekoppeltem Licht ausgebildet ist, und mit einer Lichteinkopplungsvorrichtung, einem Messbereich und einem Auskoppelbereich, die in unterschiedlichen Abschnitten des Trägermediums angeordnet sind. Die Lichteinkopplungsvorrichtung umfasst eine Lichtquelle, wobei die Lichtquelle dazu ausgebildet ist, Licht mit einer ersten Wellenlänge abzustrahlen, wobei die Lichteinkopplungsvorrichtung derart angeordnet und dazu ausgebildet ist, das Licht der ersten Wellenlänge in das Trägermedium einzukoppeln. Das Trägermedium ist ausgebildet, das eingekoppelte Licht von der Lichteinkopplungsvorrichtung mittels interner Reflexion an den Messbereich zu übertragen, wobei der Messbereich als holographisches Element mit einer ersten Beugungsstruktur ausgebildet ist, die dazu ausgelegt ist, eingekoppeltes Licht mit der ersten Wellenlänge, das auf die erste Beugungsstruktur fällt, aus dem Trägermedium auszukoppeln und Licht mit der ersten Wellenlänge, das von außerhalb des Trägermediums auf die erste Beugungsstruktur fällt, in das Trägermedium in Richtung des Auskoppelbereichs einzukoppeln.

Dabei ist die erste Beugungsstruktur ferner als Multiplex-Beugungsstruktur ausgebildet, die dazu ausgelegt ist, zusätzlich Licht in einem zweiten Wellenlängenbereich, das von außerhalb des Trägermediums auf die Beugungsstruktur fällt, in Richtung des Auskoppelbereichs einzukoppeln.

Der Auskoppelbereich ist als holographisches Element mit einer zweiten Beugungsstruktur ausgebildet ist, die als Multiplex-Beugungsstruktur ausgebildet und dazu ausgelegt ist, Licht der ersten Wellenlänge und Licht des zweiten Wellenlängenbereichs, das aus Richtung des Messbereichs auf die zweite Beugungsstruktur fällt, aus dem Trägermedium auf eine Kameravorrichtung auszukoppeln. Die Kameravorrichtung ist dazu ausgebildet, das auf die Kameravorrichtung ausgekoppelte Licht zu erfassen und in Form von Bilddaten, die mit dem erfassten Licht korrelieren, bereitzustellen.

Mit anderen Worten ist die Vorrichtung zum farbabhängigen Detektieren beziehungsweise Aufnehmen von Bildern mit einem Trägermedium zum Leiten von eingekoppeltem Licht, einer Lichteinkopplungsvorrichtung und zwei Bereichen ausgebildet, nämlich einem Messbereich und einem Auskoppelbereich, die sich in unterschiedlichen Abschnitten des Trägermediums befinden können. Das heißt, dass die Bereiche beabstandet voneinander angeordnet sind.

Das Trägermedium kann beispielsweise aus Glas und/oder Kunststoff gefertigt sein, wobei das Licht mittels interner Reflexion, das heißt Totalreflexion, innerhalb des Trägermediums übertragen werden kann. Die Farbigkeit bezieht sich hier auf die beiden genannten Lichttypen, d.h. die erste Wellenlänge und den davon verschiedenen zweiten Wellenlängenbereich.

Die Lichteinkopplungsvorrichtung der Vorrichtung umfasst eine Lichtquelle, die Licht mit einer ersten Wellenlänge abstrahlen kann, wobei die Lichtquelle insbesondere eine Fotodiode oder Laserdiode aufweisen kann. Die Lichteinkopplungsvorrichtung kann das Licht der ersten Wellenlänge beispielsweise über eine oder mehrere Linsen in das Trägermedium einkoppeln, es ist jedoch auch möglich, dass die Lichteinkopplungsvorrichtung ein holographisches Element mit einer Beugungsstruktur umfasst, die dazu ausgelegt ist, das Licht mit der ersten Wellenlänge von der Lichtquelle in das Trägermedium einzukoppeln. Beispielsweise kann die Lichteinkopplungsvorrichtung auch derart an dem Auskoppelbereich angeordnet sein, dass das holographische Element mit der zweiten Beugungsstruktur das Licht der ersten Wellenlänge in das Trägermedium einkoppeln kann. Das so eingekoppelte Licht von der Lichteinkopplungsvorrichtung kann dann innerhalb des Trägermediums an den Messbereich übertragen werden, wobei der Messbereich ein holographisches Element mit einer ersten Beugungsstruktur aufweist, die das in das Trägermedium eingekoppelte Licht mit der ersten Wellenlänge beugen kann, sodass es aus dem Trägermedium ausgekoppelt wird.

Das aus dem Messbereich ausgekoppelte Licht mit der ersten Wellenlänge kann dann verwendet werden, ein Objekt mit der ersten Wellenlänge auszuleuchten, wobei von dem Objekt reflektiertes Licht mit der ersten Wellenlänge wieder zurück in den Messbereich von der ersten Beugungsstruktur in das Trägermedium in Richtung des Auskoppelbereichs eingekoppelt werden kann. In Richtung des Auskoppelbereichs bedeutet hier eine makroskopische Richtung vom Messbereich entlang des Trägermediums zu dem Auskoppelbereich, beziehungsweise ein Richtungsvektor in die Ausbreitungsrichtung des Lichts mittels interner Reflexion. Ein Lichtpfad kann hierbei natürlich aufgrund der internen Refexion einen Zick-Zack-Verlauf aufweisen.

Ein holographisches Element, das auch als holographisch-optisches Element (HOE) bezeichnet wird, ist ein optisches Element, deren Funktionsprinzip auf Holographie beruht und das mittels holographischer Verfahren, das heißt holographischer Belichtung, hergestellt werden kann. Ein holographisches Element kann insbesondere als optisches Gitter beziehungsweise Beugungsgitter ausgebildet sein, wobei ein optisches Gitter Grundsätzlich zumindest abschnittsweise periodische Strukturen, sogenannte Gitterstrukturen, aufweisen, die durch den physikalischen Effekt der Beugung eine Lichtlenkung, wie sie zum Beispiel von Spiegeln, Linsen oder Prismen bekannt ist, herbeiführen können. Fällt Licht beziehungsweise fallen Lichtstrahlen auf das optische Gitter, wobei die einfallenden Lichtstrahlen insbesondere die Bragg-Gleichung erfüllen, werden die Lichtstrahlen durch das optische Gitter gebeugt oder abgelenkt. Die Lichtlenkung kann somit insbesondere durch Interferenzerscheinungen der durch das optische Gitter gebeugten Lichtstrahlen erfolgen.

Vorzugsweise können optische Gitter gegenüber dem einfallenden Licht winkel- beziehungsweise richtungsselektiv und/oder wellenlängen- beziehungsweise frequenzselektiv ausgebildet sein. Somit kann nur Licht, das aus einer Richtung, zum Beispiel in einem Winkelbereich von etwa 180 Grad, auf ein optisches Gitter fällt, abgelenkt werden. Licht, das aus einer anderen Richtung auf das optische Gitter fällt, wird vorzugsweise nicht abgelenkt. Zusätzlich oder alternativ kann auch nur Licht einer Wellenlänge, insbesondere eines schmalen Wellenlängenbereichs, von dem optischen Gitter in einem bestimmten Beugungswinkel abgelenkt werden. Dadurch kann beispielsweise nur ein Anteil des Licht in einem bestimmten Wellenlängen- oder Frequenzbereich von dem optischen Gitter abgelenkt werden, der übrige Anteil des Lichts kann hingegen ohne abgelenkt zu werden durch das Gitter propagieren. Von polychromatischem Licht, welches auf das optische Gitter trifft, kann somit wenigstens ein monochromatischer Lichtanteil abgespaltet werden.

Die erste Beugungsstruktur ist ferner als Multiplex-Beugungsstruktur ausgebildet, das heißt, dass ein holographisches optisches Gitter polychromatisches Licht beugen kann, insbesondere das Licht der ersten Wellenlänge und der zweiten Wellenlänge. Diese werden auch als holografische Mehrvolumenfachgitter (multiplexed volume holografic gratings, kurz: MVHG) bezeichnet und können beispielsweise durch Verändern der Periodizität der Gitterstruktur eines optischen Gitters oder durch Anordnen mehrerer holografisches Volumengitter hintereinander hergestellt werden, wodurch die Multiplex-Beugungsstruktur entsteht.

Hierdurch wird es zusätzlich ermöglicht, dass die erste Beugungsstruktur Licht in einem zweiten Wellenlängenbereich, das von außerhalb des Trägermediums auf die Beugungsstruktur fällt, in Richtung des Auskoppelbereichs eingekoppelt wird. Licht in dem zweiten Längenbereich kann beispielsweise Außenlicht umfassen, das bedeutet Umgebungslicht, das auf das aufzunehmende Objekt fallen kann. Der zweite Wellenlängenbereich kann auch nur bestimmte Abschnitte des Spektrums umfassen, beispielsweise nur eine rote, grüne oder blaue Komponente des Außenlichts. Das Licht der ersten Wellenlänge und des zweiten Wellenlängenbereichs kann dann innerhalb des Trägermediums zu dem Auskoppelbereich geleitet werden, der mit einer zweiten Beugungsstruktur ausgebildet sein kann, die wiederum als Multiplex-Beugungsstruktur ausgebildet ist und das Licht, das von außerhalb des Trägermediums in das Trägermedium eingekoppelt wurde, auf eine Kameravorrichtung auskoppeln kann.

Die Kameravorrichtung, die beispielsweise CMOS- oder CCD-Sensoren umfassen kann, insbesondere als Sensorenarray, kann das so ausgekoppelte Licht erfassen und in Form von Bilddaten aufnehmen, die mit dem erfassten Licht korrelieren. Hierzu können die Sensoren der Kameravorrichtung sensitiv für verschiedene Wellenlängen des Lichts sein, beispielsweise über Strahlteiler wie Prismen oder Gitter oder über Farbfilter beziehungsweise Farbfilteranordnungen.

Durch diese Ausführungsform ergibt sich der Vorteil, dass zusätzlich zu Umgebungslicht in einem zweiten Wellenlängenbereich eine Lichtquelle mit einer ersten Wellenlänge dazugeschaltet werden kann, wobei das Licht mit der ersten Wellenlänge aus dem Messbereich der Vorrichtung abgestrahlt werden kann, ohne dass eine Verschiebung der Position der Lichtquelle notwendig ist, wodurch keine Verschiebung der optischen Achse auftritt. Des Weiteren kann durch die holographischen Elemente Platz eingespart werden, der ansonsten für Prismen oder Gitter notwendig gewesen wäre.

Zu der Erfindung gehören auch Ausführungsformen, durch die sich zusätzliche Vorteile ergeben.

Eine Ausführungsform sieht vor, dass das Trägermedium quaderförmig ausgebildet ist und die Lichteinkopplungsvorrichtung an einer Schmalseite des Trägermediums angebracht ist und die Kameravorrichtung an einer Oberflächenseite des Trägermediums angebracht ist. Mit anderen Worten kann das Trägermedium beispielsweise eine Kante in Längsrichtung a, eine Kante in Breitenrichtung b und eine Kante in Höhenrichtung c aufweisen, wobei die Längsrichtung a vorzugsweise größer als b und c ist. Die Lichteinkopplungsvorrichtung kann an einer Schmalseite beziehungsweise Stirnseite des Trägermediums angebracht sein, das bedeutet an einer Seite beziehungsweise Fläche, die durch b und c aufgespannt wird. Die Kameravorrichtung kann an einer Oberflächenseite des Trägermediums angebracht sein, wobei eine Oberflächenseite eine Fläche ist, die durch a und b aufgespannt wird. Vorzugsweise kann vorgesehen sein, dass der Messbereich und Auskoppelbereich in Längsrichtung a zueinander versetzt angeordnet sind, wobei insbesondere eine Richtung des Messbereichs, in der das Licht ausgekoppelt wird und die Kameravorrichtung an entgegengesetzten Oberflächen beabstandet voneinander angeordnet sind.

Vorzugsweise können jedoch auch nur die gegenüberliegenden Oberflächenseiten, das heißt die Deckflächen des Trägermediums, planparallel sein oder einen gleichen Krümmungsradius aufweisen und das Licht kann entweder über eine Oberflächenseite oder eine Schmalseite eingekoppelt werden. Das bedeutet, dass zum Beispiel jede beliebige geometrische Form mit planparallelen Oberflächenseiten, beispielsweise eine zylindrische Form, vorgesehen sein kann.

Durch diese Ausführungsform ergibt sich der Vorteil, dass die Lichteinkopplungsvorrichtung und die Kameravorrichtung beabstandet voneinander ausgebildet sein können, wodurch Bauraum eingespart werden kann und eine flachere Bauweise ermöglicht wird.

Vorzugsweise ist vorgesehen, dass die erste Wellenlänge im infraroten Wellenlängenbereich liegt, insbesondere in einem Wellenlängenbereich von 800 Nanometern bis 1000 Nanometern. Hierdurch ergibt sich der Vorteil, dass ein Objekt durch Zuschalten der Lichtquelle mit der ersten Wellenlänge in dem infraroten Wellenlängenbereich ausgeleuchtet werden kann.

Vorzugsweise ist vorgesehen, dass der zweite Wellenlängenbereich im sichtbaren Wellenlängenbereich liegt, insbesondere in einem Wellenlängenbereich von 380 Nanometern bis 780 Nanometern. Hierdurch kann die Vorrichtung Umgebungslicht aufnehmen und dieses beispielsweise zusätzlich mit infrarotem Licht überlagern.

Eine Ausführungsform sieht vor, dass der Messbereich und der Auskoppelbereich direkt in das Trägermedium, insbesondere in eine Oberflächenstruktur des Trägermediums, eingearbeitet sind oder das Trägermedium als separates Element zu dem Messbereich und dem Auskoppelbereich ausgebildet ist. Im ersten Fall können der Messbereich und der Auskoppelbereich somit beispielsweise direkt in eine Oberflächenstruktur des Trägermediums eingearbeitet werden. Somit kann das Trägermedium selbst als HOE ausgebildet sein. Im zweiten Fall können der Messbereich, der Auskoppelbereich und das Trägermedium separat ausgebildet sein. Dabei können der Messbereich und der Auskoppelbereich beispielsweise wenigstens ein erstes Element bilden und das Trägermedium kann ein zweites Element bilden, welches an dem ersten Element anliegt. Somit können der Messbereich und der Auskoppelbereich in wenigstens einem HOE ausgebildet sein. Beispielsweise können der Messbereich und der Auskoppelbereich in unterschiedlichen Abschnitten einer holografischen Folie oder Platte ausgebildet sein. Zum Befestigen der Folie oder Platte an dem Trägermedium kann die Folie oder Platte an das Trägermedium angeklebt sein. Alternativ kann die holografische Folie auch als Adhäsionsfolie ausgebildet sein und direkt, also ohne Klebstoff, durch molekulare Kräfte an der Oberfläche des Trägermediums haften.

Eine weitere Ausführungsform sieht vor, dass die erste Beugungsstruktur und die zweite Beugungsstruktur als ein holographisches Volumengitter oder als ein holographisches Oberflächengitter ausgebildet sind. Besonders bevorzugt können optische Gitter mittels Belichtung eines Substrats, also beispielsweise fotolithografisch oder holografisch, hergestellt werden. In diesem Zusammenhang können die optischen Gitter dann auch als holografische oder holografisch-optische Gitter bezeichnet werden. Es sind zwei Arten von holografischoptischen Gittern bekannt: holografische Oberflächengitter (surface holografic gratings, kurz: SHG) und holografische Volumengitter (volume holografic gratings, kurz: VHG). Bei holografischen Oberflächengittern kann die Gitterstruktur durch optisches Verformen einer Oberflächenstruktur des Substrats erzeugt werden. Durch die veränderte Oberflächenstruktur kann auftreffendes Licht abgelenkt, zum Beispiel reflektiert werden. Beispiele für holografische Oberflächengitter sind sogenannte Sägezahn- oder Blazegitter. Im Gegensatz dazu kann die Gitterstruktur bei holografischen Volumengittern in das ganze Volumen oder einen Teilbereich des Volumens des Substrats eingearbeitet sein. Holografische Oberflächengitter und holografische Volumengitter sind in der Regel frequenzselektiv, können jedoch als Multiplex- beziehungsweise Mehrfachvolumengitter für mehrere Wellenlängen selektiv ausgebildet sein.

Eine weitere Ausführungsform sieht vor, dass die bereitgestellten Bilddaten eine Verknüpfung von Einzelbildern der jeweiligen erfassten Wellenlänge sind. Eine Verknüpfung von Einzelbildern der jeweiligen erfassten Wellenlänge kann beispielsweise eine mathematische Operation der jeweiligen Bilder miteinander umfassen, wie zum Beispiel eine additive Überlagerung, eine Subtraktion, ein Ergänzen von Farbbereichen der Bilder mit Bildinformationen einer jeweiligen Wellenlänge, eine Faltung und eine Filterung. Insbesondere kann vorgesehen sein, dass mittels eines Transparenzeffekts, der durch eine Anpassung des alpha-Kanals der Einzelbilder erzeugt werden kann, mehrere Ebenen dargestellt werden können, die beispielsweise durch eine erhöhte Eindringtiefe der ersten Wellenlänge aufgenommen werden kann. Durch diese Ausführungsform ergibt sich der Vorteil, dass Daten der Einzelbilder innerhalb der bereitgestellten Bilddaten übersichtlich bereitgestellt werden können.

Des Weiteren sieht die Erfindung vor, dass die Lichteinkopplungsvorrichtung eine weitere Lichtquelle umfasst, die dazu ausgebildet ist, Licht mit einer dritten Wellenlänge abzustrahlen, und wobei die Lichteinkopplungsvorrichtung derart angeordnet und dazu ausgebildet ist, Licht mit der dritten Wellenlänge in das Trägermedium einzukoppeln. Dabei ist die erste Beugungsstruktur ferner dazu ausgebildet, Licht mit der dritten Wellenlänge, das auf die erste Beugungsstruktur fällt, aus dem Trägermedium auszukoppeln und Licht mit der dritten Wellenlänge, das von außerhalb des Trägermediums auf die erste Beugungsstruktur fällt, in das Trägermedium in Richtung des Auskoppelbereichs einzukoppeln, wobei die erste Beugungsstruktur ferner als Multiplex-Beugungsstruktur ausgebildet ist, die dazu ausgelegt ist, zusätzlich Licht der dritten Wellenlänge, das von außerhalb des Trägermediums auf die Beugungsstruktur fällt, in Richtung des Auskoppelbereichs einzukoppeln. Die zweite Beugungsstruktur, die als Multiplex-Beugungsstruktur ausgebildet ist, ist ferner dazu ausgelegt, Licht mit der dritten Wellenlänge, das aus Richtung des Messbereichs auf die zweite Beugungsstruktur fällt, aus dem Trägermedium auf die Kameravorrichtung auszukoppeln. Mit anderen Worten kann die Lichteinkopplungsvorrichtung eine weitere Lichtquelle umfassen, die Licht mit einer dritten Wellenlänge abstrahlt und in das Trägermedium einkoppeln kann, wobei die erste Beugungsstruktur des Messbereichs das Licht der dritten Wellenlänge aus dem Trägermedium aus- und wieder einkoppeln kann und die zweite Beugungsstruktur des Auskoppelbereichs das Licht der dritten Wellenlänge auskoppeln kann, sodass die Kameravorrichtung das ausgekoppelte Licht erfassen kann. Durch diese Ausführungsform ergibt sich der Vorteil, dass eine Lichtquelle mit einer weiteren Wellenlänge dazugeschaltet werden kann, um ein weiteres Merkmal eines angeleuchteten Objekts sichtbar zu machen.

Vorzugsweise ist vorgesehen, dass die dritte Wellenlänge im ultravioletten Wellenlängenbereich liegt, insbesondere in einem Wellenlängenbereich zwischen 180 Nanometern bis 380 Nanometern. Hierbei kann insbesondere vorgesehen sein, dass das Trägermedium UV-lichtdurchlässig ist und dieses leiten kann, beispielsweise kann das Trägermedium hauptsächlich Quarzglas beinhalten.

Eine Ausführungsform sieht vor, dass die Vorrichtung ferner eine Identifikationseinrichtung aufweist, die dazu ausgebildet ist, die Bilddaten der Kameravorrichtung zu empfangen und die Bilddaten auf ein vorgegebenes biometrisches Identifikationsmerkmal hin zu überprüfen und bei Eintreten einer vorbestimmten Vergleichsbedingung ein Steuersignal zu erzeugen. Mit anderen Worten kann eine Identifikationseinrichtung vorgesehen sein, die die Bilddaten der Kameravorrichtung empfängt und mit einem vorgegebenen biometrischen Identifikationsmerkmal vergleicht, wobei bei einer vorbestimmten Vergleichsbedingung ein Steuersignal erzeugt werden kann. Die Identifikationseinrichtung kann beispielsweise ein Prozessor sein, der Bilddaten miteinander vergleichen kann, insbesondere ein vorgegebenes biometrisches Identifikationsmerkmal, das in einer Datenbank der Identifikationseinrichtung gespeichert sein kann. Bei Eintreten der vorbestimmten Vergleichsbedingung, mittels der beispielsweise eine Ähnlichkeit der Bilddaten mit dem vorgegebenen biometrischen Identifikationsmerkmal festgestellt werden kann, kann dann ein Steuersignal erzeugt werden. Das vorgegebene biometrische Identifikationsmerkmal kann beispielsweise eine Oberflächenstruktur oder eine Pigmentstruktur einer Haut einer Person, wie beispielsweise eines Fingers, eine Linienstruktur oder eine biometrische Struktur, wie zum Beispiel eine Venenstruktur in der Haut, unter der Oberfläche einer Hand eines Benutzers oder einer Iris eines Auges eines Benutzers umfassen. Die vorbestimmte Vergleichsbedingung kann beispielsweise umfassen, dass eine Mustererkennung eine Übereinstimmung von über 90 Prozent ergibt. Durch diese Ausführungsform ergibt sich der Vorteil, dass die Bilddaten auf ein vorgegebenes biometrisches Identifikationsmerkmal hin überprüft werden können und bei einer Übereinstimmung ein Steuersignal zum Ansteuern weiterer Geräte erzeugt werden kann.

Ein weiterer Aspekt der Erfindung betrifft ein Kraftfahrzeug mit einer Vorrichtung nach einer der vorhergehenden Ausführungsformen, wobei die Vorrichtung in einer Fensterscheibe des Kraftfahrzeugs integriert ist und das Steuersignal einen Öffnungsmechanismus des Kraftfahrzeugs ansteuert. Das Ansteuern des Öffnungsmechanismus kann dabei vorzugsweise ein Öffnen eines Schlosses einer Fahrzeugtür des Kraftfahrzeugs umfassen. Durch diese Ausführungsform ergibt sich der Vorteil, dass das Kraftfahrzeug schlüssellos geöffnet werden kann. Das erfindungsgemäße Kraftfahrzeug ist bevorzugt als Kraftwagen, insbesondere als Personenkraftwagen oder Lastkraftwagen, oder als Personenbus ausgestaltet.

Eine Ausführungsform des Kraftfahrzeugs sieht vor, dass die Vorrichtung in einem Bildschirm und/oder einer Armlehne des Kraftfahrzeugs integriert ist und das Steuersignal eine Startvorrichtung des Kraftfahrzeugs ansteuert. Durch diese Ausführungsform ergibt sich der Vorteil, dass das Kraftfahrzeug schlüssellos von einem berechtigten Benutzer gestartet werden kann.

Erfindungsgemäß ist auch eine Rechenvorrichtung mit einer Vorrichtung nach einer vorhergehenden Ausführungsform bereitgestellt, wobei die Vorrichtung in einem Bildschirm der Rechenvorrichtung integriert ist und das Steuersignal einen Zugriff auf die Rechenvorrichtung freigibt. Die Rechenvorrichtung kann insbesondere ein Computer, ein Tablet-PC und ein Smartphone umfassen. Durch diese Ausführungsform ergibt sich der Vorteil, dass die Rechenvorrichtung durch das vorgegebene biometrische Identifikationsmerkmal eines berechtigten Benutzers freigegeben werden kann.

Zu der Erfindung gehören auch Weiterbildungen der erfindungsgemäßen Vorrichtung, die Merkmale aufweisen, wie sie bereits im Zusammenhang mit den Weiterbildungen des erfindungsgemäßen Kraftfahrzeugs beschrieben worden sind. Aus diesem Grund sind die entsprechenden Weiterbildungen der erfindungsgemäßen Vorrichtung hier nicht noch einmal beschrieben.

Die Erfindung umfasst auch die Kombinationen der Merkmale der beschriebenen Ausführungsformen.

Im Folgenden sind Ausführungsbeispiele der Erfindung beschrieben. Hierzu zeigt:
- Fig. 1: eine schematische Seitenansicht einer Vorrichtung gemäß einer beispielhaften Ausführungsform;
- Fig. 2: eine Darstellung einer Innenseite einer Kraftfahrzeugtür gemäß einer beispielhaften Ausführungsform;
- Fig. 3: eine Darstellung einer Außenseite einer Kraftfahrzeugtür gemäß einer beispielhaften Ausführungsform;
- Fig. 4: eine Darstellung einer Rechenvorrichtung in einem Kraftfahrzeug gemäß einer beispielhaften Ausführungsform.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden. Daher soll die Offenbarung auch andere als die dargestellten Kombinationen der Merkmale der Ausführungsformen umfassen. Des Weiteren sind die beschriebenen Ausführungsformen auch durch weitere der bereits beschriebenen Merkmale der Erfindung ergänzbar.

In den Figuren bezeichnen gleiche Bezugszeichen jeweils funktionsgleiche Elemente.

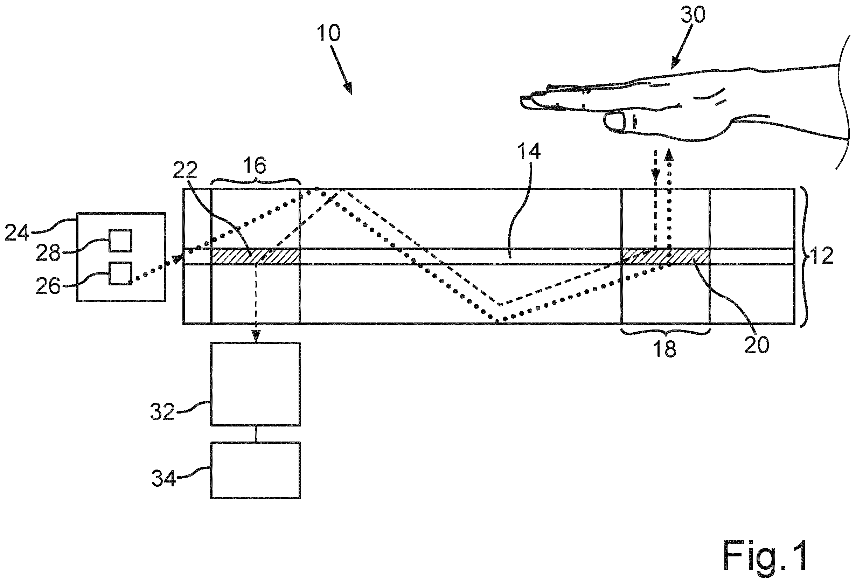

In Fig. 1 ist eine schematische Darstellung einer Vorrichtung 10 zum farbabhängigen Detektieren von Bildinhalten dargestellt. Die Vorrichtung 10 umfasst ein Trägermedium 12, welches als Lichtleiter zum Übertragen von eingekoppeltem Licht durch interne Reflexion ausgebildet ist.

Das Trägermedium 12 kann quaderförmig ausgebildet sein, beispielsweise mit separaten quaderförmigen Elementen, also Platten, die zum Bilden des Trägermediums in einer Sandwichbauweise aufgebaut sind. Beispielsweise kann das Trägermedium 12 zwei Glasplatten umfassen, die als Lichtleiter dienen und die Deckschichten des Trägermediums bilden. Der Kern des Trägermediums, der von den beiden Glasplatten eingeschlossen wird, kann ein holographisches Element 14 aufweisen, welches beispielsweise als transparente Fotopolymerfolie ausgebildet sein kann. Die Glasplatten liegen mit einer jeweiligen Oberfläche direkt an jeweils gegenüberliegenden Oberflächen des holographischen Elements an. Anders ausgedrückt, liegen das holographische Element 14 und die Glasplatten flächig mit ihren jeweiligen von einer Längs- und Breitseite eingeschlossenen Flächen aneinander an. Zusätzlich zu der Lichtleitung können die Glasplatten das holographische Element 14 auch vor äußeren Umwelteinflüssen schützen. Fig. 1 zeigt insbesondere als Schnittbild der Vorrichtung zum farbabhängigen Detektieren von Bildinhalten, bei dem die Vorrichtung 10 mit einem Schnitt entlang einer Längsachse dargestellt ist.

Das Trägermedium 12 kann einen Auskoppelbereich 16 und einen Messbereich 18 umfassen, die in unterschiedlichen Abschnitten des Trägermediums angeordnet sind und beispielsweise entlang einer Längserstreckungsrichtung des Trägermediums versetzt angeordnet sind. Das holographische Element 14 kann im Auskoppelbereich 16 und im Messbereich 18 für den jeweiligen Bereich mittels Holographieverfahren derart belichtet worden sein, dass sich eine erste Beugungsstruktur 20 im Messbereich und eine zweite Beugungsstruktur 22 im Auskoppelbereich bilden kann, die insbesondere als ein holographisches Volumengitter oder als ein holographisches Oberflächengitter ausgebildet sein kann. Das bedeutet, dass die erste und zweite Beugungsstruktur 20, 22 eine Gitterstruktur aufweisen, die Licht mit einer vorgegebenen Wellenlänge in einem vorbestimmten Winkel beugen kann. Der Messbereich und der Auskoppelbereich können dabei direkt in das Trägermedium eingearbeitet sein oder die Bereiche können als separates Element zu dem Trägermedium ausgebildet sein.

Die Vorrichtung 10 umfasst des Weiteren eine Lichteinkopplungsvorrichtung 24, die an einer Schmalseite beziehungsweise Stirnseite des Trägermediums angebracht sein kann. Die Lichteinkopplungsvorrichtung 24 kann vorzugsweise eine Infrarotlichtquelle 26 und eine UV-Lichtquelle 28 umfassen, die je nach Anwendungsfall für die Vorrichtung 10 hinzugeschaltet werden können.

Die Infrarotlichtquelle 26 kann dabei Licht mit einer ersten Wellenlänge abstrahlen, die im infraroten Wellenlängenbereich liegt, also beispielsweise in einem Wellenlängenbereich von 800 bis 1000 Nanometern. Vorzugsweise kann die Infrarotlichtquelle 26 eine Fotodiode aufweisen, die Licht mit einer Wellenlänge von 850 Nanometern abstrahlt. Die UV-Lichtquelle 28 kann Licht mit einer dritten Wellenlänge abstrahlen, wobei die dritte Wellenlänge im ultravioletten Wellenlängenbereich liegt, beispielsweise zwischen 180 bis 380 Nanometern. Vorzugsweise kann auch die UV-Lichtquelle 28 als eine Leuchtdiode ausgebildet sein, die Licht mit einer Wellenlänge von 340 Nanometern abstrahlt. Die hier für die Fotodioden angegebenen Wellenlängen sind Spitzenwellenlängen ("peak wavelenght"), die einen Bereich angeben, an dem das Spektrum der Leuchtdiode die höchste Intensität erreicht. Dem Fachmann ist hierbei bekannt, dass diese Spitzenwellenlänge um einige Nanometer abweichen kann, beispielsweise um +/- 30 Nanometer.

Die Lichteinkopplungsvorrichtung 24 an der Stirnseite des Trägermediums 12 ist ferner dazu ausgebildet, das Licht der Lichtquellen in das Trägermedium 12 einzukoppeln. Hierzu kann beispielsweise ein Linsensystem (nicht gezeigt) vorgesehen sein, das einer Einkopplungsbedingung beziehungsweise Resonanzbedingung für das Trägermedium 12 genügt und das Licht in das Trägermedium 12 einkoppeln kann, sodass es innerhalb des Trägermediums mittels Totalreflexion übertragen werden kann.

In diesem Ausführungsbeispiel strahlt die Infrarotlichtquelle 26 Licht mit der ersten Wellenlänge in das Trägermedium 12 ab, was durch eine durchgezogene Linie in Fig. 1 dargestellt ist. Das Licht mit der ersten Wellenlänge wird innerhalb des Trägermediums mittels interner Reflexion zu dem Messbereich weitergeleitet, wo es dann auf die erste Beugungsstruktur fällt und gebeugt. Dadurch kann das Licht der ersten Wellenlänge aus dem Trägermedium zum Belichten eines Objekts 30 ausgekoppelt werden.

In diesem Ausführungsbeispiel kann das zu messende Objekt 30 eine Hand eines Benutzers sein und es kann beispielsweise vorgesehen sein, ein Handvenenmuster zu bestimmen. Hierzu ist Licht im infraroten Wellenlängenbereich besonders geeignet, da mittels infraroten Lichts eine Handvenenmustererkennung nach bekannten Verfahren durchgeführt werden. Hierbei kann das infrarote Licht, das aus dem Trägermedium 12 auf die Hand 30 abgestrahlt wird, reflektiert werden und wieder zurück in den Messbereich auf die erste Beugungsstruktur 20 fallen.

Zusätzlich zu dem infraroten Licht kann auch Umgebungslicht, das von der Hand 30 gestreut wurde, in den Messbereich 18 eintreten und auf die erste Beugungsstruktur 20 fallen, was als gestrichelte Linie angedeutet ist. Das Umgebungslicht kann in einem zweiten Wellenlängenbereich liegen, wobei der zweite Wellenlängenbereich im sichtbaren Wellenlängenbereich von vorzugsweise 380 Nanometern bis 780 Nanometern liegen kann.

Die erste Beugungsstruktur 20 kann ferner als Multiplex-Beugungsstruktur ausgebildet sein, die Licht einer vorbestimmten Wellenlänge in einem vorbestimmten Winkel beugen kann und somit zurück in das Trägermedium 12 einkoppeln kann. Die Multiplex-Beugungsstruktur kann dabei eine verschränkte Beugungsstruktur umfassen, wobei mehrere optische Gitter, beispielsweise in Form von Volumengittern, ineinander erzeugt werden, indem die bei der Erzeugung der Multiplex-Beugungsstruktur das holographische Element mehrfach belichtet wird, um mehrere Gitterstrukturen zu erzeugen, wobei eine jeweilige Gitterstruktur Licht mit einer anderen Wellenlänge in einem vorbestimmten Winkel beugen kann. Alternativ oder zusätzlich kann auch vorgesehen sein, dass mehrere holographische Elemente in einer Schicht- beziehungsweise Sandwichbauweise vorgesehen sind, die über einen Transferkleber miteinander verbunden sind, wobei jede Schicht durch geeignete Belichtungsverfahren für eine jeweilige Wellenlänge ausgebildet ist. Beispielsweise können drei aufeinander geklebte holographische Elemente vorgesehen sein, wobei zum Beispiel jeweils ein holographisches Element sensitiv für einen infraroten, ultravioletten und sichtbaren Wellenlängenbereich ist.

Das so in das Trägermedium 12 eingekoppelte Licht kann dann in Richtung des Auskoppelbereichs geleitet werden, wo es auf die zweite Beugungsstruktur 22 trifft, die wiederum als Multiplex-Beugungsstruktur ausgebildet sein kann und dazu ausgelegt sein kann, das Licht aus Richtung des Messbereichs aus dem Trägermedium auf eine Kameravorrichtung 32 auszukoppeln.

Die Kameravorrichtung 32 kann insbesondere einen Fotodetektor wie beispielsweise einen CCD-Detektor oder CMOS-Detektor aufweisen, der insbesondere als ein Detektorarray zum Aufnehmen von Bilddaten ausgebildet sein kann. Hierbei können die Bilddaten in Korrelation mit dem erfassten Licht bereitgestellt werden. Die Kameravorrichtung 32 kann dabei vorzugsweise an einer Oberflächenseite des Trägermediums angebracht sein.

Die durch die Kameravorrichtung 32 bereitgestellten Bilddaten können vorzugsweise eine Verknüpfung von Einzelbildern der jeweiligen erfassten Wellenlänge aufweisen, wodurch sich in diesem Ausführungsbeispiel beispielsweise zum einen ein Bild der Hand 30 ergibt, das mit einem Handvenenmuster aus dem Einzelbild der infraroten Wellenlänge überlagert werden kann. Die so erfassten Bilddaten können dann von einer Identifikationseinrichtung 34 empfangen werden, die beispielsweise ein Computerprozessor sein kann.

Die Identifikationseinrichtung 34 kann dann die Bilddaten auf ein vorgegebenes biometrisches Identifikationsmerkmal hin überprüfen und bei Eintreten einer vorbestimmten Vergleichsbedingung ein Steuersignal erzeugen. In diesem Ausführungsbeispiel kann das vorgegebene biometrische Identifikationsmerkmal das Handvenenmuster umfassen, wobei die Identifikationseinrichtung 34 beispielsweise das Muster der aufgenommenen Handvenen mit einem vorgegebenen, das heißt in der Identifikationseinrichtung gespeicherten, Muster vergleicht und bei einer Übereinstimmung von beispielsweise über 90 Prozent ein Steuersignal erzeugen kann, mit dem weitere Geräte angesteuert werden können.

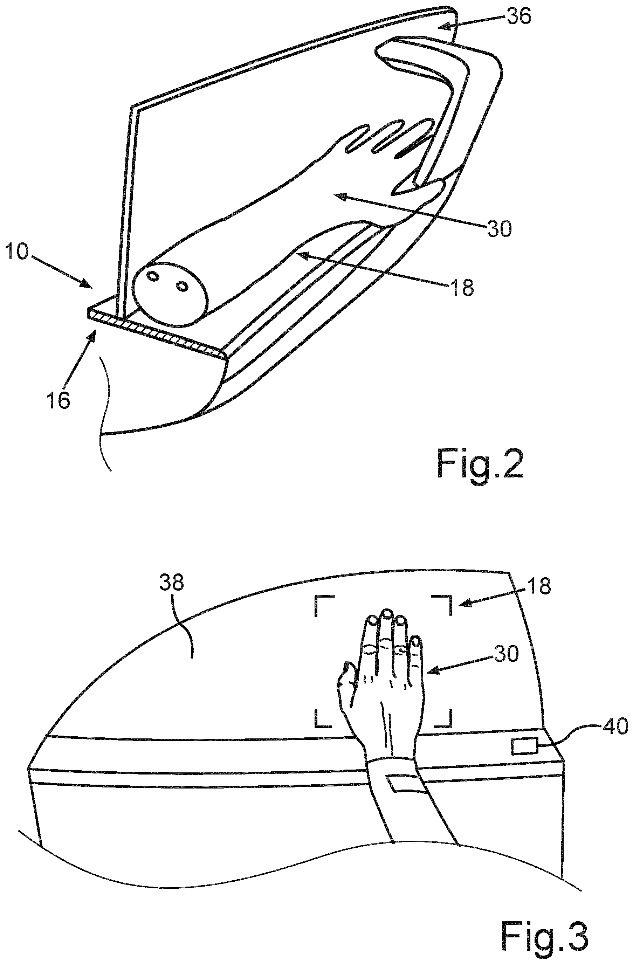

In Fig. 2 ist eine Innenseite eines Kraftfahrzeugs 36, insbesondere einer Kraftfahrzeugtür, gemäß einer beispielhaften Ausführungsform dargestellt. Die Vorrichtung 10 zum farbabhängigen Detektieren von Bildinhalten kann in dieser Ausführungsform beispielsweise in einer Auflage der Kraftfahrzeugtür vorgesehen sein. Ein Benutzer kann hier beispielsweise zur Verifikation seiner Identität seine Hand 30 auf den Messbereich 18 der Vorrichtung 10 legen, wobei mittels zuvor beschriebener Verfahren das reflektierte Licht von der Hand 30 in den Auskoppelbereich 16 geleitet werden kann, der beispielsweise in der Türverkleidung untergebracht sein kann. Zusätzlich können in der Türverkleidung auch die Kameravorrichtung 32 und die Identifikationseinrichtung 34 vorgesehen sein, wobei die Identifikationseinrichtung 34 nach erfolgter Überprüfung beispielsweise ein Steuersignal an eine Startvorrichtung (nicht gezeigt) des Kraftfahrzeugs senden kann, die einen Motor des Kraftfahrzeugs starten kann.

In Fig. 3 ist eine weitere beispielhafte Ausführungsform der Vorrichtung 10 dargestellt. In diesem Ausführungsbeispiel kann die Vorrichtung 10 in einer Fensterscheibe 38 des Kraftfahrzeugs integriert sein. Bei Auflegen der Hand 30 des Benutzers auf den Messbereich 18 kann dann beispielsweise ein Bild des Handvenenmusters erfasst und von der Identifikationseinrichtung 34 überprüft werden. Bei Eintreten der vorbestimmten Vergleichsbedingung kann dann zum Beispiel ein Steuersignal an einen Öffnungsmechanismus 40 der Kraftfahrzeugtür gesendet werden, die beispielsweise ein Schloss der Fahrzeugtür öffnen kann.

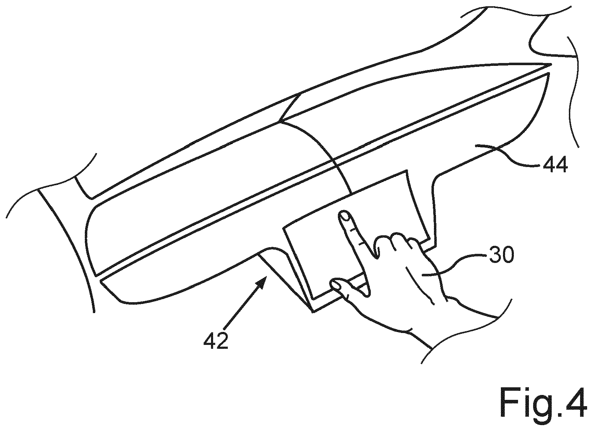

In Fig. 4 ist eine Darstellung einer Rechenvorrichtung 42 gemäß einer beispielhaften Ausführungsform gezeigt. Die Rechenvorrichtung 42 ist in diesem Ausführungsbeispiel in einem Armaturenbrett 44 eines Kraftfahrzeugs integriert. Insbesondere kann die Rechenvorrichtung 42 ein Infotainmentsystem des Kraftfahrzeugs sein. Die Rechenvorrichtung 42 kann vorzugsweise einen Bildschirm aufweisen, wobei beispielsweise ein Glas, insbesondere ein Schutzglas, des Bildschirms in dieser Ausführungsform der Messbereich 18 der Vorrichtung 10 zum farbabhängigen Detektieren von Bildinhalten sein kann. Bei Auflegen oder Annähern der Hand 30 kann hier beispielsweise die zuvor beschriebene Handvenenmustererkennung stattfinden und es kann beispielsweise ein Zugriff auf die Rechenvorrichtung 42 bei erfolgreicher Identifikation freigegeben werden.

In einer anderen beispielhaften Ausführungsform besteht ein Aspekt darin, dass beispielsweise eine Annäherung einer Hand zur Handvenenerkennung an den Messbereich zur Aufnahme ausreichend ist. Das heißt, dass die Hand nicht auf den Messbereich 18 aufgelegt werden muss, wodurch eine Verschmutzung des Messbereichs beziehungsweise eine Verschmutzung der Hand vermieden werden kann.

Eine weitere Anwendungsmöglichkeit wäre, die Vorrichtung in einen Schaltknauf, ein Lenkrad, einen Rückspiegel, eine Mittelkonsolenoberfläche und/oder ein Dachfenster des Kraftfahrzeugs zu integrieren, um eine Identifikation eines Benutzers zu ermöglichen.

Insgesamt zeigen die Beispiele, wie durch die Erfindung ein farbabhängiges Detektieren von Bildinhalten und eine biometrische Identifikation durch Handvenenerkennung über ein holographisches Element bereitgestellt werden kann.

Weitere beispielhafte Ausführungsformen können folgenden Aspekten umfassen:
Aspekt 1: Vorrichtung (10) zum farbabhängigen Detektieren von Bildinhalten mit einem Trägermedium (12), welches als Lichtleiter zum Übertragen von eingekoppeltem Licht ausgebildet ist, und einer Lichteinkopplungsvorrichtung (24), einem Messbereich (18) und einem Auskoppelbereich (16), die in unterschiedlichen Abschnitten des Trägermediums angeordnet sind, wobei
   - die Lichteinkopplungsvorrichtung (24) eine Lichtquelle (26) umfasst, wobei die Lichtquelle (26) dazu ausgebildet ist, Licht mit einer ersten Wellenlänge abzustrahlen, und wobei die Lichteinkopplungsvorrichtung (24) derart angeordnet und dazu ausgebildet ist, Licht mit der ersten Wellenlänge in das Trägermedium (12) einzukoppeln;
   - das Trägermedium (12) ausgebildet ist, das eingekoppelte Licht von der Lichteinkopplungsvorrichtung mittels interner Reflexion an den Messbereich (18) zu übertragen;
   - der Messbereich (18) als holographisches Element (14) mit einer ersten Beugungsstruktur (20) ausgebildet ist, die dazu ausgelegt ist, eingekoppeltes Licht mit der ersten Wellenlänge, das auf die erste Beugungsstruktur (20) fällt, aus dem Trägermedium (12) auszukoppeln und Licht mit der ersten Wellenlänge, das von außerhalb des Trägermediums auf die erste Beugungsstruktur (20) fällt, in das Trägermedium (12) in Richtung des Auskoppelbereichs (16) einzukoppeln, wobei die erste Beugungsstruktur (20) ferner als Multiplex-Beugungsstruktur ausgebildet ist, die dazu ausgelegt ist, zusätzlich Licht in einem zweiten Wellenlängenbereich, das von außerhalb des Trägermediums auf die erste Beugungsstruktur (20) fällt, in Richtung des Auskoppelbereichs einzukoppeln;
   - der Auskoppelbereich (16) als holographisches Element (14) mit einer zweiten Beugungsstruktur (22) ausgebildet ist, die als Multiplex-Beugungsstruktur ausgebildet und dazu ausgelegt ist, Licht der ersten Wellenlänge und Licht des zweiten Wellenlängenbereichs, das aus Richtung des Messbereichs auf die zweite Beugungsstruktur (22) fällt, aus dem Trägermedium (12) auf eine Kameravorrichtung (32) auszukoppeln; wobei
   - die Kameravorrichtung (32) dazu ausgebildet ist, das auf die Kameravorrichtung (32) ausgekoppelte Licht zu erfassen und in Form von Bilddaten, die mit dem erfassten Licht korrelieren, bereitzustellen.
Aspekt 2: Vorrichtung (10) nach Aspekte 1, wobei das Trägermedium quaderförmig ausgebildet ist und die Lichteinkopplungsvorrichtung (24) an einer Schmalseite des Trägermediums angebracht ist und die Kameravorrichtung an einer Oberflächenseite des Trägermediums angebracht ist.
Aspekt 3: Vorrichtung (10) nach einem der vorhergehenden Aspekte, wobei die erste Wellenlänge im infraroten Wellenlängenbereich liegt, insbesondere in einem Wellenlängenbereich von 800 nm bis 1000 nm.
Aspekt 4: Vorrichtung (10) nach einem der vorhergehenden Aspekte, wobei der zweite Wellenlängenbereich im sichtbaren Wellenlängenbereich liegt, insbesondere in einem Wellenlängenbereich von 380 nm bis 780 nm.
Aspekt 5: Vorrichtung (10) nach einem der vorhergehenden Aspekte, wobei der Messbereich (18) und der Auskoppelbereich (16) direkt in das Trägermedium (12), insbesondere in eine Oberflächenstruktur des Trägermediums, eingearbeitet sind oder das Trägermedium (12) als separates Element zu dem Messbereich (18) und dem Auskoppelbereich (16) ausgebildet ist.
Aspekt 6: Vorrichtung (10) nach einem der vorhergehenden Aspekte, wobei die erste Beugungsstruktur (20) und die zweite Beugungsstruktur (22) als ein holographisches Volumengitter oder als ein holographisches Oberflächengitter ausgebildet sind.
Aspekt 7: Vorrichtung (10) nach einem der vorhergehenden Aspekte, wobei die bereitgestellten Bilddaten eine Verknüpfung von Einzelbildern der jeweiligen erfassten Wellenlänge sind.
Aspekt 8: Vorrichtung (10) nach einem der vorhergehenden Aspekte, wobei die Lichteinkopplungsvorrichtung (24) eine weitere Lichtquelle (28) umfasst, die dazu ausgebildet ist, Licht mit einer dritten Wellenlänge abzustrahlen, und wobei die Lichteinkopplungsvorrichtung (24) derart angeordnet und dazu ausgebildet ist, Licht mit der dritten Wellenlänge in das Trägermedium (12) einzukoppeln; wobei
   die erste Beugungsstruktur (20) ferner dazu ausgebildet ist, Licht mit der dritten Wellenlänge das auf die erste Beugungsstruktur (20) fällt, aus dem Trägermedium (12) auszukoppeln und Licht mit der dritten Wellenlänge, das von außerhalb des Trägermediums auf die erste Beugungsstruktur (20) fällt, in das Trägermedium in Richtung des Auskoppelbereichs einzukoppeln, wobei die erste Beugungsstruktur (20) ferner als Multiplex-Beugungsstruktur ausgebildet ist, die dazu ausgelegt ist, zusätzlich Licht der dritten Wellenlänge, das von außerhalb des Trägermediums auf die Beugungsstruktur fällt, in Richtung des Auskoppelbereichs einzukoppeln; und
   die zweite Beugungsstruktur (22), die als Multiplex-Beugungsstruktur ausgebildet ist, ferner dazu ausgelegt ist, Licht mit der dritten Wellenlänge, das aus Richtung des Messbereichs auf die zweite Beugungsstruktur fällt, aus dem Trägermedium (12) auf die Kameravorrichtung (32) auszukoppeln.
Aspekt 9: Vorrichtung (10) nach Aspekt 8, wobei die dritte Wellenlänge im ultravioletten Wellenlängenbereich liegt, insbesondere in einem Wellenlängenbereich zwischen 180 nm bis 380 nm.
Aspekt 10: Vorrichtung (10) nach einem der vorhergehenden Aspekte, wobei die Vorrichtung (10) ferner eine Identifikationseinrichtung (34) aufweist, die dazu ausgebildet ist, die Bilddaten der Kameravorrichtung zu empfangen und die Bilddaten auf ein vorgegebenes biometrisches Identifikationsmerkmal hin zu überprüfen und bei Eintreten einer vorbestimmten Vergleichsbedingung ein Steuersignal zu erzeugen.
Aspekt 11: Kraftfahrzeug (36) mit einer Vorrichtung nach Aspekt 10, wobei die Vorrichtung (10) in einer Fensterscheibe (38) des Kraftfahrzeugs integriert ist und das Steuersignal ein Öffnungsmechanismus (40) des Kraftfahrzeugs ansteuert.
Aspekt 12: Kraftfahrzeug (36) mit einer Vorrichtung (10) nach Aspekt 10, wobei die Vorrichtung (10) in einem Bildschirm und/oder einer Armlehne des Kraftfahrzeugs integriert ist und das Steuersignal eine Startvorrichtung des Kraftfahrzeugs ansteuert.
Aspekt 13: Rechenvorrichtung (42) mit einer Vorrichtung (10) nach Aspekt 10, wobei die Vorrichtung (10) in einem Bildschirm der Rechenvorrichtung integriert ist und das Steuersignal einen Zugriff auf die Rechenvorrichtung (42) freigibt.

## Patentansprüche

1. Vorrichtung (10) zum farbabhängigen Detektieren von Bildinhalten mit einem Trägermedium (12), welches als Lichtleiter zum Übertragen von eingekoppeltem Licht ausgebildet ist, und einer Lichteinkopplungsvorrichtung (24), einem Messbereich (18) und einem Auskoppelbereich (16), die in unterschiedlichen Abschnitten des Trägermediums angeordnet sind, wobei
- die Lichteinkopplungsvorrichtung (24) eine Lichtquelle (26) umfasst, wobei die Lichtquelle (26) dazu ausgebildet ist, Licht mit einer ersten Wellenlänge abzustrahlen, und wobei die Lichteinkopplungsvorrichtung (24) derart angeordnet und dazu ausgebildet ist, Licht mit der ersten Wellenlänge in das Trägermedium (12) einzukoppeln;
- das Trägermedium (12) ausgebildet ist, das eingekoppelte Licht von der Lichteinkopplungsvorrichtung mittels interner Reflexion an den Messbereich (18) zu übertragen;
- der Messbereich (18) als holographisches Element (14) mit einer ersten Beugungsstruktur (20) ausgebildet ist, die dazu ausgelegt ist, eingekoppeltes Licht mit der ersten Wellenlänge, das auf die erste Beugungsstruktur (20) fällt, aus dem Trägermedium (12) auszukoppeln und Licht mit der ersten Wellenlänge, das von außerhalb des Trägermediums auf die erste Beugungsstruktur (20) fällt, in das Trägermedium (12) in Richtung des Auskoppelbereichs (16) einzukoppeln, wobei die erste Beugungsstruktur (20) ferner als Multiplex-Beugungsstruktur ausgebildet ist, die dazu ausgelegt ist, zusätzlich Licht in einem zweiten Wellenlängenbereich, das von außerhalb des Trägermediums auf die erste Beugungsstruktur (20) fällt, in Richtung des Auskoppelbereichs einzukoppeln;
- der Auskoppelbereich (16) als holographisches Element (14) mit einer zweiten Beugungsstruktur (22) ausgebildet ist, die als Multiplex-Beugungsstruktur ausgebildet und dazu ausgelegt ist, Licht der ersten Wellenlänge und Licht des zweiten Wellenlängenbereichs, das aus Richtung des Messbereichs auf die zweite Beugungsstruktur (22) fällt, aus dem Trägermedium (12) auf eine Kameravorrichtung (32) auszukoppeln; wobei
- die Kameravorrichtung (32) dazu ausgebildet ist, das auf die Kameravorrichtung (32) ausgekoppelte Licht zu erfassen und in Form von Bilddaten, die mit dem erfassten Licht korrelieren, bereitzustellen, **dadurch gekennzeichnet, dass**
die Lichteinkopplungsvorrichtung (24) eine weitere Lichtquelle (28) umfasst, die dazu ausgebildet ist, Licht mit einer dritten Wellenlänge abzustrahlen, und wobei die Lichteinkopplungsvorrichtung (24) derart angeordnet und dazu ausgebildet ist, Licht mit der dritten Wellenlänge in das Trägermedium (12) einzukoppeln; wobei
die erste Beugungsstruktur (20) ferner dazu ausgebildet ist, Licht mit der dritten Wellenlänge das auf die erste Beugungsstruktur (20) fällt, aus dem Trägermedium (12) auszukoppeln und Licht mit der dritten Wellenlänge, das von außerhalb des Trägermediums auf die erste Beugungsstruktur (20) fällt, in das Trägermedium in Richtung des Auskoppelbereichs einzukoppeln, wobei die erste Beugungsstruktur (20) ferner als Multiplex-Beugungsstruktur ausgebildet ist, die dazu ausgelegt ist, zusätzlich Licht der dritten Wellenlänge, das von außerhalb des Trägermediums auf die Beugungsstruktur fällt, in Richtung des Auskoppelbereichs einzukoppeln; und
die zweite Beugungsstruktur (22), die als Multiplex-Beugungsstruktur ausgebildet ist, ferner dazu ausgelegt ist, Licht mit der dritten Wellenlänge, das aus Richtung des Messbereichs auf die zweite Beugungsstruktur fällt, aus dem Trägermedium (12) auf die Kameravorrichtung (32) auszukoppeln.

2. Vorrichtung (10) nach Anspruch 1, wobei das Trägermedium quaderförmig ausgebildet ist und die Lichteinkopplungsvorrichtung (24) an einer Schmalseite des Trägermediums angebracht ist und die Kameravorrichtung an einer Oberflächenseite des Trägermediums angebracht ist.

3. Vorrichtung (10) nach einem der vorhergehenden Ansprüche, wobei die erste Wellenlänge im infraroten Wellenlängenbereich liegt, insbesondere in einem Wellenlängenbereich von 800 nm bis 1000 nm.

4. Vorrichtung (10) nach einem der vorhergehenden Ansprüche, wobei der zweite Wellenlängenbereich im sichtbaren Wellenlängenbereich liegt, insbesondere in einem Wellenlängenbereich von 380 nm bis 780 nm.

5. Vorrichtung (10) nach einem der vorhergehenden Ansprüche, wobei der Messbereich (18) und der Auskoppelbereich (16) direkt in das Trägermedium (12), insbesondere in eine Oberflächenstruktur des Trägermediums, eingearbeitet sind oder das Trägermedium (12) als separates Element zu dem Messbereich (18) und dem Auskoppelbereich (16) ausgebildet ist.

6. Vorrichtung (10) nach einem der vorhergehenden Ansprüche, wobei die erste Beugungsstruktur (20) und die zweite Beugungsstruktur (22) als ein holographisches Volumengitter oder als ein holographisches Oberflächengitter ausgebildet sind.

7. Vorrichtung (10) nach einem der vorhergehenden Ansprüche, wobei die bereitgestellten Bilddaten eine Verknüpfung von Einzelbildern der jeweiligen erfassten Wellenlänge sind.

8. Vorrichtung (10) nach einem der vorhergehenden Ansprüche, wobei die dritte Wellenlänge im ultravioletten Wellenlängenbereich liegt, insbesondere in einem Wellenlängenbereich zwischen 180 nm bis 380 nm.

9. Vorrichtung (10) nach einem der vorhergehenden Ansprüche, wobei die Vorrichtung (10) ferner eine Identifikationseinrichtung (34) aufweist, die dazu ausgebildet ist, die Bilddaten der Kameravorrichtung zu empfangen und die Bilddaten auf ein vorgegebenes biometrisches Identifikationsmerkmal hin zu überprüfen und bei Eintreten einer vorbestimmten Vergleichsbedingung ein Steuersignal zu erzeugen.

10. Kraftfahrzeug (36) mit einer Vorrichtung nach Anspruch 9, wobei die Vorrichtung (10) in einer Fensterscheibe (38) des Kraftfahrzeugs integriert ist und das Steuersignal ein Öffnungsmechanismus (40) des Kraftfahrzeugs ansteuert.

11. Kraftfahrzeug (36) mit einer Vorrichtung (10) nach Anspruch 9, wobei die Vorrichtung (10) in einem Bildschirm und/oder einer Armlehne des Kraftfahrzeugs integriert ist und das Steuersignal eine Startvorrichtung des Kraftfahrzeugs ansteuert.

12. Rechenvorrichtung (42) mit einer Vorrichtung (10) nach Anspruch 9, wobei die Vorrichtung (10) in einem Bildschirm der Rechenvorrichtung integriert ist und das Steuersignal einen Zugriff auf die Rechenvorrichtung (42) freigibt.

## Claims

1. A device (10) for color-dependent detection of image contents with a carrier medium (12), which is formed as a light guide for transferring coupled-in light, and a light coupling-in device (24), a measurement area (18) and a coupling-out area (16), which are arranged in different sections of the carrier medium, wherein
- the light coupling-in device (24) includes a light source (26), wherein the light source (26) is formed to radiate light with a first wavelength, and wherein the light coupling-in device (24) is arranged and formed to couple light with the first wavelength into the carrier medium (12);
- the carrier medium (12) is formed to transfer the coupled-in light from the light coupling-in device to the measurement area (18) by means of internal reflection;
- the measurement area (18) is formed as a holographic element (14) with a first diffraction structure (20), which is adapted to couple coupled-in light with the first wavelength, which is incident on the first diffraction structure (20), out of the carrier medium (12) and to couple light with the first wavelength, which is incident on the first diffraction structure (20) from outside of the carrier medium, into the carrier medium (12) towards the coupling-out area (16), wherein the first diffraction structure (20) is further formed as a multiplex diffraction structure, which is adapted to additionally couple in light in a second wavelength range, which is incident on the first diffraction structure (20) from outside of the carrier medium, towards the coupling-out area;
- the coupling-out area (16) is formed as a holographic element (14) with a second diffraction structure (22), which is formed as a multiplex diffraction structure and is adapted to couple light of the first wavelength and light of the second wavelength range, which is incident on the second diffraction structure (22) from the direction of the measurement area, out of the carrier medium (12) onto a camera device (32); wherein
- the camera device (32) is formed to capture the light coupled out onto the camera device (32) and to provide it in the form of image data, which correlates with the captured light,
**characterized in that**
the light coupling-in device (24) includes a further light source (28), which is formed to radiate light with a third wavelength, and wherein the light coupling-in device (24) is arranged and formed to couple light with the third wavelength into the carrier medium (12); wherein
the first diffraction structure (20) is further formed to couple light with the third wavelength, which is incident on the first diffraction structure (20), out of the carrier medium (12) and to couple light with the third wavelength, which is incident on the first diffraction structure (20) from outside of the carrier medium, into the carrier medium towards the coupling-out area, wherein the first diffraction structure (20) is further formed as a multiplex diffraction structure, which is adapted to additionally couple in light of the third wavelength, which is incident on the diffraction structure from outside of the carrier medium , towards the coupling-out area; and
the second diffraction structure (22), which is formed as a multiplex diffraction structure, is further adapted to couple light with the third wavelength, which is incident on the second diffraction structure from the direction of the measurement area, out of the carrier medium (12) onto the camera device (32).

2. The device (10) according to claim 1, wherein the carrier medium is cuboidally formed and the light coupling-in device (24) is attached to a narrow side of the carrier medium and the camera device is attached to a surface side of the carrier medium.

3. The device (10) according to any one of the preceding claims, wherein the first wavelength is in the infrared wavelength range, in particular in a wavelength range from 800 nm to 1000 nm.

4. The device (10) according to any one of the preceding claims, wherein the second wavelength range is in the visible wavelength range, in particular in a wavelength range from 380 nm to 780 nm.

5. The device (10) according to any one of the preceding claims, wherein the measurement area (18) and the coupling-out area (16) are directly incorporated into the carrier medium (12), in particular into a surface structure of the carrier medium, or the carrier medium (12) is formed as a separate element to the measurement area (18) and the coupling-out area (16).

6. The device (10) according to any one of the preceding claims, wherein the first diffraction structure (20) and the second diffraction structure (22) are formed as a holographic volume grating or as a holographic surface grating.

7. The device (10) according to any one of the preceding claims, wherein the provided image data is a correlation of frames of the respective captured wavelength.

8. The device (10) according to any one of the preceding claims, wherein the third wavelength is in the ultraviolet wavelength range, in particular in a wavelength range between 180 nm and 380 nm.

9. The device (10) according to any one of the preceding claims, wherein the device (10) further comprises an identification device (34), which is formed to receive the image data of the camera device and to examine the image data for a preset biometric identification feature, and to generate a control signal upon occurrence of a predetermined comparison condition.

10. A motor vehicle (36) with a device according to claim 9, wherein the device (10) is integrated in a window pane (38) of the motor vehicle and the control signal controls an opening mechanism (40) of the motor vehicle.

11. The motor vehicle (36) with a device (10) according to claim 9, wherein the device (10) is integrated in a screen and/or an armrest of the motor vehicle and the control signal controls a start device of the motor vehicle.

12. A computing device (42) with a device (10) according to claim 9, wherein the device (10) is integrated in a screen of the computing device and the control signal enables access to the computing device (42).

## Revendications

1. Dispositif (10) pour détecter des contenus d'image en fonction de la couleur, comportant un support (12) formé comme un conduit de lumière pour transmettre de la lumière couplée, et un dispositif de couplage de lumière (24), une région de mesure (18) et une région de découplage (16) qui sont agencées dans différentes parties du support,
- le dispositif de couplage de lumière (24) comprenant une source de lumière (26), la source de lumière (26) étant configurée pour émettre de la lumière à une première longueur d'onde, et le dispositif de couplage de lumière (24) étant agencé et configuré pour coupler la lumière à la première longueur d'onde dans le support (12) ;
- le support (12) étant configuré pour transmettre à la région de mesure (18), par réflexion interne, la lumière couplée provenant du dispositif de couplage de lumière,
- la région de mesure (18) étant formée comme un élément holographique (14) ayant une première structure de diffraction (20) conçue pour découpler la lumière couplée ayant la première longueur d'onde, qui est incidente sur la première structure de diffraction (20), depuis l'extérieur du support (12), et pour coupler la lumière à la première longueur d'onde, qui est incidente sur la première structure de diffraction (20) depuis l'extérieur du support, dans le support (12) en direction de la région de découplage (16), la première structure de diffraction (20) étant en outre configurée comme une structure de diffraction multiplexée conçue pour coupler en plus la lumière à une seconde gamme de longueurs d'onde, qui est incidente sur la première structure de diffraction (20) depuis l'extérieur du support, en direction de la région de découplage ;
- la région de découplage (16) étant configurée comme un élément holographique (14) avec une seconde structure de diffraction (22) configurée comme une structure de diffraction multiplexée et conçue pour découpler la lumière à la première longueur d'onde et la lumière à la seconde gamme de longueurs d'onde, qui est incidente sur la seconde structure de diffraction (22) et qui provient de la direction de la région de mesure, à partir du support (12) vers un dispositif à caméra (32) ;
- le dispositif à caméra (32) étant configuré pour capturer la lumière découplée vers le dispositif à caméra (32) et pour fournir des données d'image qui sont corrélées avec la lumière capturée,
**caractérisé en ce que**
le dispositif de couplage de lumière (24) comprend une source de lumière supplémentaire (28) configurée pour émettre de la lumière à une troisième longueur d'onde, et le dispositif de couplage de lumière (24) étant configuré pour coupler la lumière à la troisième longueur d'onde dans le support (12) ;
la première structure de diffraction (20) étant en outre configurée pour découpler la lumière à la troisième longueur d'onde, qui est incidente sur la première structure de diffraction (20), depuis l'extérieur du support (12), et pour coupler la lumière à la troisième longueur d'onde, qui est incidente sur la première structure de diffraction (20) depuis l'extérieur du support, dans le support en direction de la région de découplage, la première structure de diffraction (20) étant en outre configurée comme une structure de diffraction multiplexée conçue pour coupler en plus la lumière à la troisième longueur d'onde, qui est incidente sur la structure de diffraction depuis l'extérieur du support, en direction de la région de découplage ; et
la seconde structure de diffraction (22) formée comme une structure de diffraction multiplexée, étant en outre conçue pour découpler la lumière à la troisième longueur d'onde, qui est incidente sur la seconde structure de diffraction depuis la direction de la région de mesure, depuis l'extérieur du support (12) vers le dispositif à caméra (32).

2. Dispositif (10) selon la revendication 1, dans lequel le support est configuré en forme de parallélépipède et le dispositif de couplage de lumière (24) est placé sur un petit côté du support et le dispositif à caméra est monté sur un côté de surface du support.

3. Dispositif (10) selon l'une des revendications précédentes, dans lequel la première longueur d'onde se situe dans la gamme de longueurs d'onde de l'infrarouge, en particulier dans une gamme de longueurs d'onde de 800 nm à 1 000 nm.

4. Dispositif (10) selon l'une des revendications précédentes, dans lequel la seconde longueur d'onde se situe dans la gamme de longueurs d'onde visibles, en particulier dans une gamme de longueurs d'onde de 380 nm à 780 nm.

5. Dispositif (10) selon l'une des revendications précédentes, dans lequel la région de mesure (18) et la région de découplage (26) sont directement intégrées dans le support (12), en particulier dans une structure de surface du support, ou le support (12) est formé comme un élément séparé de la région de mesure (18) et de la région de découplage (16).

6. Dispositif (10) selon l'une des revendications précédentes, dans lequel la première structure de diffraction (20) et la seconde structure de diffraction (22) sont formées comme un réseau holographique volumique ou comme un réseau holographique surfacique.

7. Dispositif (10) selon l'une des revendications précédentes, dans lequel les données d'image fournies sont une combinaison d'images individuelles de la longueur d'onde capturée respective.

8. Dispositif (10) selon l'une des revendications précédentes, dans lequel la troisième longueur d'onde se situe dans la gamme de longueurs d'onde de l'ultraviolet, en particulier dans une gamme de longueurs d'onde comprise entre 180 nm et 380 nm.

9. Dispositif (10) selon l'une des revendications précédentes, le dispositif (10) comporte en outre un dispositif d'identification (34) configuré pour recevoir les données d'image du dispositif à caméra et pour contrôler une caractéristique d'identification biométrique prédéfinie des données d'image et pour générer un signal de commande lorsqu'une condition de comparaison prédéfinie est satisfaite.

10. Véhicule à moteur (36) comportant un dispositif selon la revendication 9, dans lequel le dispositif (10) est intégré dans une vitre (38) du véhicule à moteur et le signal de commande commande un mécanisme d'ouverture (40) du véhicule à moteur.

11. Véhicule à moteur (36) comportant un dispositif (10) selon la revendication 9, dans lequel le dispositif (10) est intégré dans un écran et/ou un accoudoir du véhicule à moteur et le signal de commande commande un dispositif de démarrage du véhicule à moteur.

12. Dispositif de calcul (42) comportant un dispositif (10) selon la revendication 9, dans lequel le dispositif (10) est intégré dans un écran du dispositif de calcul et le signal de commande permet un accès au dispositif de calcul (42).
